# EUROPEAN PATENT APPLICATION

(11) **EP 1 570 851 A1**
(43) Date of publication of application: **07.09.2005**
(21) Application number: 03777342.1
(22) Date of filing: 08.12.2003
(51) Int. Cl.: A61K 31/734, A61K 7/00, A61K 7/48, A61K 31/66, A61K 31/197, A61P 17/00, A61P 17/04

(54) **SKIN MATERIAL FOR EXTERNAL USE AND ANTIPRUTIRIC AGENT FOR EXTERNAL USE AND WRINKLE-REDUCING INSTRUMENT USING THE SAME**

(30) Priority: 10.12.2002 JP 2002358433
(71) Applicant: Tanaka, Masaya, Kobe-shi, Hyogo 654-0036 (JP)
(72) Inventor: Tanaka, Masaya, Kobe-shi, Hyogo 654-0036 (JP)
(74) Representative: Sajda, Wolf E.
(86) International application number: PCT/JP2003/015659
(87) International publication number: WO 2004/052378

(57) **Abstract**

A skin composition for external use containing an acidic composition (A) which contains an alginate(s), at least one of weak acid selected from among phosphoric acid-type weak acids and ethylenediaminetetraacetic acid-type weak acids, and water; and a solidifying/gelling agent (B) containing a calcium salt(s) which is to be supplied to the acidic composition (A) sticking to the skin surface. In some cases, the skin composition for external use further includes an absorbent to be impregnated with the acidic composition (A).

## Description

### Technical Field

The present invention relates to a skin composition for external use principally providing an immediate antipruritic effect and also providing a wrinkle-flattening effect by employing an absorbent, as well as an antipruritic agent for external use and a wrinkle-flattening instrument using the same.

### Background Art

Heretofore, a variety of skin compositions for external use have been proposed, which use an alginate and a calcium salt in combination. Examples of the proposed materials include: (1) a wound covering material including a container containing a sodium alginate solution and a container containing an aqueous solution containing a metal ion whose valency is 2 or more (see, Japanese Unexamined Patent Publication No. 11-178910); and (2) a face pack product including a composition for topical application onto skin containing a water-soluble alginate and a composition containing a water-soluble salt of alkaline earth metal that works to solidify the composition for topical application onto skin (see Japanese Unexamined Patent Publication No. 2002-128636).

These wound covering material and face pack product utilize that a solution or suspension of alginate(s) is gelatinized by calcium ions. However, these products do not employ a weak acid and are not reported to exert the antipruritic effect. Furthermore, it has not been reported that these products offer the wrinkle-flattening effect by using an absorbent.

By the way, pruritus is known to be associated with insect bites, eczema, heat rash and the like or with senile pruritus. It is generally said that as any kind of pruritus persists longer, discomfort becomes more severe. This is undesirable in terms of mental health. In this connection, there has been a strong demand for the development of a technology for immediately ceasing itching sensation. On the other hand, facial wrinkles at the outer corners of the eyes or on the forehead are a serious problem for women in terms of facial beauty. Hence, there has long been a strong demand for the development of a technology for readily removing the facial wrinkles.

In view of the foregoing, the present invention has been accomplished. It is an object of the invention to provide a skin composition for external use capable of providing an immediate antipruritic effect, as well as an antipruritic agent for external use using the same. It is another object of the present invention to provide a skin composition for external use, which employs an absorbent for readily exerting a wrinkle-flattening effect, as well as a wrinkle-flattening instrument using the same.

### Disclosure of the Invention

The present inventor has made various studies on acidic polysachharides containing alginates. In the study process, the inventor has discovered that an immediate antipruritic effect heretofore unknown to the art is produced by using an acidic composition (A) containing: an alginate(s), at least one weak acid selected from among phosphoric acid-type weak acids and ethylenediaminetetraacetic acid-type weak acids, and water, in combination with a solidifying/gelling agent (B) containing a calcium salt(s). Thus, the invention has been accomplished. The inventor has also discovered that a wrinkle-flattening effect is produced by using an absorbent capable of being impregnated with the acidic composition (A), in addition to the acidic composition (A) and the solidifying/gelling agent (B). Thus, the invention has been accomplished in another aspect.

A skin composition for external use of the present invention is characterized by comprising the following acidic composition (A) and the following solidifying/gelling agent (B) which is to be supplied to the acidic composition (A) sticking to the skin:
(A) an acidic composition containing: an alginate(s), at least one weak acid selected from among phosphoric acid-type weak acids and ethylenediaminetetraacetic acid-type weak acids, and water;
(B) a solidifying/gelling agent containing a calcium salt(s).

According to the above composition, when the acidic composition (A) is applied onto skin surface and then, the solidifying/gelling agent (B) is applied onto the acidic composition, for example, the solidifying/gelling agent (B) permeates into the acidic composition (A) to gelatinize and solidify the alginate(s), so that the immediate antipruritic effect is obtained. The skin composition for external use of the present invention contains an alginate(s) as the acidic polysaccharide component, and a weak acid selected from the group of phosphoric acid and ethylenediaminetetraacetic acid as the acidic component and hence, the material offers advantages in providing aesthetic effects to improve skin texture, to tighten facial skin, to whiten skin, to impart skin clearness, to moisturize skin and to lighten blemishes, or in accelerating the penetration of topical agents, such as medicinal products and cosmetics, into skin.

It is noted here that the "skin composition for external use" of the present invention is defined to include not only the above dermatological agent(s) comprising the aforesaid acidic composition (A) and solidifying/gelling agent (B) but also a mode using an absorbent additionally to these acidic composition (A) and solidifying/gelling agent (B).

In the above skin composition for external use, at least one selected from the group of sodium alginate and potassium alginate may preferably be used as the alginate. In this case, the resultant acidic composition (A) has an advantage of exhibiting high affinity for skin surface. Furthermore, the acidic composition has another advantage of forming a more solid gel more quickly through reaction with the solidifying/gelling agent (B).

It is preferred to use at least one selected from the group of sodium dihydrogen phosphate and potassium dihydrogen phosphate as the above weak acid of phosphoric acid-type weak acids. In this case, the compound has an advantage of providing a quicker antipruritic effect as compared with sodium ethylenediaminetetraacetate or the like as the weak acid of ethylenediaminetetraacetic acid-type weak acids. Furthermore, the compound also has an advantage of further enhancing the aforesaid aesthetic effects and medical effect.

It is preferred to use at least one selected from the group of calcium chloride and calcium glycerophosphate as the above calcium salt. The use of calcium chloride offers an advantage that calcium chloride, having a high utilization rate of calcium ions, instantaneously forms a solid gel upon contact with the acidic composition (A). On the other hand, the use of calcium glycerophosphate has an advantage of causing less irritation to the skin or eyes.

It is preferred to use an aqueous solution containing calcium glycerophosphate and lactic acid as the aforesaid solidifying/gelling agent (B). In this case, lactic acid promotes the dissolution of insoluble calcium glycerophosphate. In addition, the acidity is not excessively increased and hence, the acidic composition (A) is prone to solidify. Furthermore, the skin or eye irritation caused from the strong acidity of the solidifying/gelling agent (B) can be suppressed.

The aforesaid acidic composition (A) may preferably contain an adhesive(s) for enhancing the affinity for skin surface. In this case, the stickiness of the composition to the skin is further increased so that the composition does not drip off from an itching area, thus the antipruritic effect to the affected area is ensured.

Alternatively, the aforesaid skin composition for external use may further comprise an absorbent in which the above acidic composition is impregnated additionally to the above acidic composition (A) and solidifying/gelling agent (B). The above absorbent is used to make the acidic composition adhere to the skin surface by direct application onto the skin surface. In this case, when the absorbent impregnated with the acidic composition is applied to a wrinkled area and the solidifying/gelling agent (B) is supplied thereto from above, the gelation of the composition proceeds so rapidly that the wrinkles can be reduced readily. Since the use of an absorbent involves the use of the acidic composition (A) and the solidifying/gelling agent (B), the immediate antipruritic effect can also be obtained. What is more, the aforementioned aesthetic effects and the like can also be obtained.

Particularly, in a case where a material shaped like a face mask for substantially covering the overall facial area is used as the above absorbent, the material is capable of readily flattening the facial wrinkles at the outer corners of the eyes or on the forehead, which are the serious problem for women in terms of facial beauty.

An antipruritic agent for external use comprising the aforesaid skin composition for external use exerts an immediate antipruritic effect regardless of the types of itches such as associated with insect bites and eczema.

A wrinkle-flattening instrument comprising the skin composition for external use including the above absorbent, or a face mask-type wrinkle-flattening instrument comprising the above face mask-shaped absorbent exerts an immediate wrinkle-flattening effect.

### Brief Description of the Drawings

FIG. 1 is a group of schematic diagrams illustrating one example of the use of an antipruritic agent for external use according to one embodiment of the skin composition for external use of the present invention; and
FIG. 2 is a group of schematic diagrams illustrating one example of the use of a wrinkle-flattening instrument according to one embodiment of the skin composition for external use of the present invention.

### Best Modes for Carrying Out the Invention

The preferred embodiments of the skin composition for external use of the present invention will be described by way of examples of antipruritic agents for external use principally directed to the antipruritic effect and wrinkle-flattening instruments principally directed to the wrinkle-flattening effect.

### Antipruritic Agent for External Use

An antipruritic agent for external use according to the embodiment of the present invention includes: an acidic composition (A) containing an alginate(s), at least one weak acid selected from among phosphoric acid-type weak acids and ethylenediaminetetraacetic acid-type weak acids, and water; and a solidifying/gelling agent (B) containing a calcium salt(s).

The alginate constituting the acidic composition (A) is not particularly limited so long as the alginate forms a solid gel through reaction with a calcium salt(s). Examples of a usable alginate include; alkaline metal salts such as sodium salt or potassium salt of alginic acid; organic amine salts such as ammonium salt of alginic acid, triethylamine salt and triethanolamine salt of alginic acid; basic amino acid salts such as alginic salt and lysine salt of alginic acid. These salts may be used alone or in combination of plural types. Above all, alkaline metal salts of alginic acid are more preferred for the reason of providing an acidic composition (A) having good sense of use. Sodium alginate and potassium alginate are further preferred because of the reasons that the resultant acidic composition (A) has high affinity for the skin surface, and that such a salt more quickly forms a more solid gel through reaction with the solidifying/gelling agent (B).

The preferable concentration of an alginate in the acidic composition (A) is 0.1 wt% or more. If the concentration is less than 0.1 wt%, the solid gel may not be formed even if the solidifying/gelling agent (B) contains the calcium salt(s) in sufficient concentrations. There is no particular upper limit of the concentration of alginate so long as the alginate is used in such a concentration as to provide an acidic composition (A) which is easily applied onto the itching area.

The weak acid of phosphoric acid-type weak acid constituting the acidic composition (A) contains phosphoric acid and normally has a pH in the range of 4.0 or more and less than 7.0 in a 1 wt% aqueous solution. Examples of a usable weak phosphoric acid-type weak acid include sodium dihydrogen phosphate and potassium dihydrogen phosphate. On the other hand, the weak acid of ethylenediaminetetraacetic acid-type weak acids contains ethylenediaminetetraacetic acid and normally has a pH in the range of 4.0 or more and less than 7.0 in a 1 wt% aqueous solution. Examples of a usable ethylenediaminetetraacetic acid include tetrasodium ethylenediaminetetraacetate, trisodium ethylenediaminetetraacetate, disodium ethylendiamine-tetraacetate and the like. Above all, potassium dihydrogen phosphate and sodium dihydrogen phosphate are more preferred because these compounds assuredly offers a more rapid antipruritic effect and higher aesthetic and medical effects as compared with the weak acids of ethylendiaminetetraacetic acid-type weak acids. The aforementioned weak acids may be used alone or in combination of plural types.

Besides the aforementioned weak acids, also usable are inorganic acids such as phosphoric acid, boric acid and carbonic acid; and organic acids including carboxylic acids such as formic acid, acetic acid, acrylic acid, benzoic acid, oxalic acid, malonic acid, glutaric acid, adipic acid, pimelic acid and phthalic acid; oxycarboxylic acids such as lactic acid; amino acids such as glycin, alanine, valine, leucine, tyrosine, threonine, cystine, thyroxine, asparagine, glutamine, asparaginic acid and glutamic acid; and the like.

The concentration of weak acid such as phosphoric acid-type weak acids or ethylenediaminetetraacetic acid-type weak acids may preferably be present in the acidic composition (A) in such concentrations as to quickly form the solid gel through reaction with the solidifying/gelling agent (B) to be described hereinlater, but not to fail to achieve the effects of the present invention by causing the acidic composition (A) itself to solidify. In a case where sodium dihydrogen phosphate is used, for example, the acidic composition (A) may preferably contain the sodium dihydrogen phosphate in concentrations of 0.1 to 5.0 wt%.

The water constituting the acidic composition (A) is not particularly limited and may include natural water, tap water and purified water such as ion-exchanged water, membrane filtered water, and the like.

In addition to an alginate(s), a specific weak acid(s) and water, the acidic composition (A) may be further admixed with any of the known additives such as adhesives, preservatives, moisturizers, surfactants, oils, perfumes, dyes, UV absorbing/reflecting agents, physiologically active materials and the like so far as it (they) does not impair the effects of the antipruritic agent for external use according to the present embodiment. In a case where an adhesive is used for enhancing the affinity for skin, in particular, the acidic composition (A) applied onto the itching area is prevented from dripping off, thus assuredly exerting the antipruritic effect on the target area.

Usable as adhesives are naturally occurring polymers, semi-synthetic polymers, synthetic polymers and inorganic substances that are exemplified as follows. The adhesives may be used alone or in combination of plural types.

Naturally occurring polymers: plant derived polymers such as gum arabic, carrageenan, galactan, agar, quince seed, guar gum, tragacanth, pectin, mannan, locust bean gum, rice starch, wheat starch, corn starch and potato starch; microbial polymers such as curdlan, xanthan gum, succinoglucan, dextran, hyaluronic acid and pullulan; protein-type polymers such as albumin, casein, collagen, gelatin and fibroin.

Semi-synthetic polymers: cellulose-type polymers such as ethyl cellulose, processed starch, carboxymethyl cellulose and salts thereof, carboxymethylethyl cellulose and salts thereof, carboxymethyl starch and salts thereof, croscarmellose and salts thereof, crystalline cellulose, acetylcellulose, cellulose acetate phthalate, hydroxyethyl cellulose, hydroxypropyl starch, hydroxypropyl cellulose, hydroxypropylmethyl cellulose, hydroxypropylmethyl cellulose phthalate, powdery cellulose, methyl cellulose, methylhydroxypropyl cellulose; starch-derived polymers such as pregelatinized starch, partially pregelatinized starch, carboxymethyl starch, dextrin and methyl starch; alginate-derived polymers such as propylene glycol alginate; other polysaccharide-derived polymers such as sodium chondroitin sulfate and sodium hyaluronate; and the like.

Synthetic polymers: carboxyvinyl polymers, sodium polyacrylate, polyvinylacetal diethylaminoacetate, polyvinyl alcohol, polyvinyl pyrrolidone, methacrylic acid-ethylacrylate copolymer, methacrylic acid-ethylmethacrylate copolymer, ethylmethacrylate-trimethylammoniumethyl chloride methacrylate copolymer, dimethylaminoethyl methacrylate-methylmethacrylate copolymer and the like.

Inorganic substances: hydrated silicon dioxide, light anhydrous silicic acid, colloidal alumina, bentonite, laponite and the like.

In a case where an adhesive is used, the adhesive may preferably be present in the acidic composition (A) in concentrations of 0.1 to 5.0 wt%. If the concentration is less than 0.1 wt%, the affinity for the skin may not be enhanced. Conversely if the concentration exceeds 5.0 wt%, the gelation rate may be decreased.

Such an acidic composition (A) may be prepared by, for example, adding an alginate(s), a specific weak acid(s) and, as required, other additive(s) to water with mixing and stirring.

The solidifying/gelling agent (B) used in combination with such an acidic composition (A) and acting to gelatinize the alginate(s) contains a calcium salt(s).

Examples of a usable calcium salt include: calcium chloride, calcium glycerophosphate, calcium gluconate, calcium lactate, calcium phosphate, calcium citrate, calcium hydrogen phosphate, anhydrous dibasic calcium phosphate and the like. These salts may be used alone or in combination of plural types. Above all, water-soluble calcium salts are preferred because the salts in solid state such as of powder or granule can supply calcium ions by the water of the acidic composition (A) when they are directly dispersed to it. Calcium chloride is particularly preferred because calcium chloride has high utilization rate of calcium ions and is capable of forming a solid gel more quickly. In a case where it is desired to suppress the skin or eye irritation, calcium glycerophosphate may preferably be used. Incidentally, those calcium salts, such as calcium citrate and calcium hydrogen phosphate, having low solubility in neutral water but increased solubility in acidic water, or those calcium salts, such as anhydrous dibasic calcium phosphate, which are insoluble in neutral water but dissolve in acidic water may be used in the form of acidic solution using any of various inorganic acids and/or organic acids.

The amount or concentration of a calcium salt varies depending upon the solubility of the calcium salt, the proportion of calcium in the calcium salt, the concentration of the alginate in the acidic composition (A) used, and the like. Normally, the amount or concentration of a calcium salt is properly decided such that the calcium salt can accomplish the gelation/solidification of the acidic composition (A) within 10 seconds or so.

In a case where a calcium chloride aqueous solution is used as the solidifying/gelling agent (B), calcium chloride may preferably be present in concentrations of 0.1 to 10 wt%. If the concentration is less than 0.1 wt%, the acidic composition (A) may not form a solid gel. Conversely if the concentration exceeds 10 wt%, the gelation rate may not be increased any more and besides, a skin irritation and the like may be caused.

In a case where a calcium glycerophosphate aqueous solution is used as the solidifying/gelling agent (B), it is preferred to add an acid(s) because calcium glycerophosphate has such a low solubility to be slightly soluble in water. The existence of an acid(s) increases the solubility of calcium glycerophosphate in water so that the concentration of calcium ions in the solution is increased. Any of an organic acid or an inorganic acid can be used as an acid(s). Among others, lactic acid is preferred from the viewpoint of taste and irritation. The solidifying/gelling agent (B) may preferably contain calcium glycerophosphate in concentrations of 1 to 5 wt% and an acid in concentrations of 0.2 to 2 wt%. If the concentration of calcium glycerophosphate is less than 1 wt% and the acid concentration is less than 0.2 wt%, the concentration of calcium ions is so low that the acidic composition (A) may not form a solid gel. On the other hand, if the concentration of calcium glycerophosphate exceeds 5 wt% and the acid concentration exceeds 2 wt%, the gelation rate may not be increased any more and besides, it may cause skin and eye irritation, and unpleasant taste when it is inside the mouse.

In addition to the calcium salt(s), the solidifying/gelling agent (B) may be further admixed with any of the known additives such as perfumes and dyes so long as such an additive does not impair the effects of the antipruritic agent for external use of the present embodiment.

In a case where the solidifying/gelling agent (B) is in liquid form, the agent can be prepared by, for example, adding the calcium salt(s) in a solvent, such as water, with mixing and stirring.

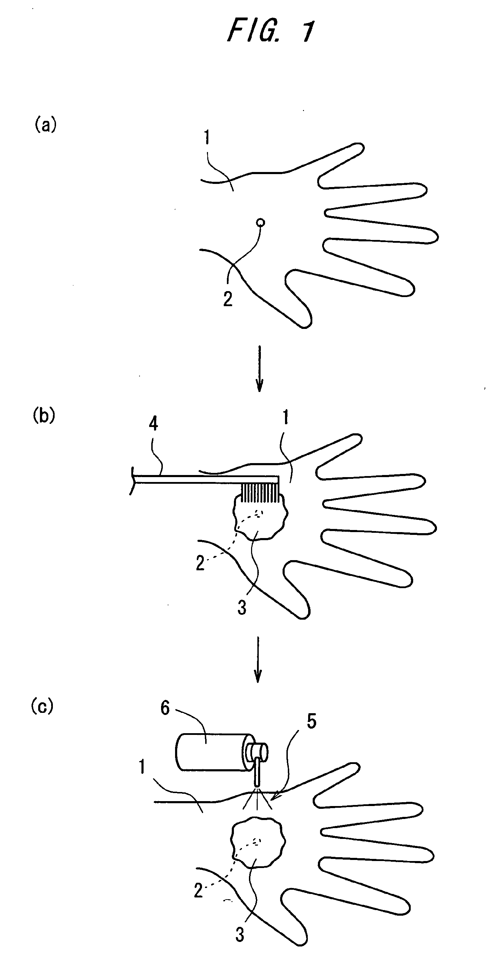

The antipruritic agent for external use according to the present embodiment may be used in the following manner, for example. Specifically, as shown in FIG. 1, an amount of acidic composition (A) 3 is first placed on an itching area 2 on the dorsum of hand 1 and then, is spread with a brush 4 or the like so as to form a coating having a suitable thickness (see FIG. 1A and FIG. 1B). Subsequently, a solidifying/gelling agent (B) 5 is supplied onto the coating of the acidic composition (A) 3 by spraying from a spray can 6 (see FIG. 1C). Thus is obtained the immediate antipruritic effect because the contact surface with the itching area 2 is moisturized while a solid gel is quickly formed on the surface of the acidic composition (A). In addition, the aesthetic effects such as whitening can also be provided because the acidic composition contains an alginate(s) as the acidic polysaccharide, and at least one weak acid selected from among phosphoric acid-type weak acids and ethylenediaminetetraacetic acid-type weak acids as the acid component.

The application of the acidic composition (A) is not limited to the above method and may be sprayed by means of a spray can or the like, for example.

On the other hand, the supply of the solidifying/gelling agent (B) is not limited to the above method. For instance, the solidifying/gelling agent (B) may be placed on the palm and then be applied onto the coating of the acidic composition (A). Alternatively, the solidifying/gelling agent (B) may be impregnated into an absorbent such as unwoven fabric, which may be brought into contact with the coating of the acidic composition (A) for transferring the solidifying/gelling agent (B). In a case where the solidifying/gelling agent (B) is in solid form, the agent may be dispersed on the coating of the acidic composition (A). In an alternative approach, the solidifying/gelling agent (B) may be encapsulated in microcapsules, which may be dispersed in the acidic composition (A). The resultant dispersion may be painted on the itching area and the microcapsules may be crushed for supplying the solidifying/gelling agent (B).

In this use, the acidic composition (A) may preferably be applied in a thickness of 0.1 to 2.0 mm. If the coating thickness is less than 0.1 mm, a portion of the acidic composition (A) that is in direct contact with the skin surface also forms a solid gel so quickly that an adequate antipruritic effect may not be exerted. Conversely, if the coating thickness exceeds 2.0 mm, a sufficiently solidified gel may not be formed on the overall applied area and hence, the adequate antipruritic effect may not be exerted.

In this use, the ratio between the acidic composition (A) and the solidifying/gelling agent (B) is not particularly limited so long as a sufficient amount of solidifying/gelling agent (B) is delivered to the overall acidic composition (A) stuck to the skin surface.

While the foregoing description pertains to the antipruritic agent for external use which includes the acidic composition (A) and the solidifying/gelling agent (B), the absorbent to be described hereinlater may also be used, for example. As impregnated with the acidic composition (A), the absorbent may be applied to the itching area. Then, the solidifying/gelling agent (B) may be supplied onto the absorbent (antipruritic agent for external use). In this case as well, the antipruritic effect can be obtained. In this case, an absorbent is used so that the wrinkle-flattening effect, which is described hereinlater, can also be obtained.

The antipruritic agent for external use according to the present embodiment has a primary object to obtain the antipruritic effect. Therefore, the agent preferably adopts the combination of an aqueous solution, as the acidic composition (A), containing sodium alginate, a weak acid of phosphoric acid-type weak acids (particularly sodium dihydrogen phosphate, potassium dihydrogen phosphate) and water; and an aqueous mixture solution of calcium chloride or a solution mixture containing calcium glycerophosphate and lactic acid, as the solidifying/gelling agent (B).

### Wrinkle-Flattening Instrument

A wrinkle-flattening instrument according to the present embodiment includes: an acidic composition (A) containing an alginate(s), at least one weak acid selected from among phosphoric acid-type weak acids and ethylenediaminetetraacetic acid-type weak acids, and water; a solidifying/gelling agent (B) containing a calcium salt(s); and an absorbent to be impregnated with the acidic composition (A).

The absorbent is not particularly limited so long as the absorbent is capable of being impregnated with the acidic composition (A). The absorbent may take any of various forms such as fabrics, unwoven fabrics, sponge and the like. Above all, the unwoven fabric is preferred because this material is light and crease-resistant. A material for the absorbent may be any of natural fibers, synthetic fibers and semi-synthetic fibers. Examples of the natural fiber include: plant-derived fibers such as from cotton and hemp; and animal-derived fibers such as wool, silk, sponge and the like. Examples of the synthetic fiber include: nylon, vinylon, Tetron, acryl, polyester and the like. Examples of the semi-synthetic fiber include: regenerated fibers such as viscose rayon (hereinafter, simply referred to as "rayon"); acetate rayon; and the like. These fiber materials may be used alone or in combination of plural types.

The absorbent may preferably have a thickness in the range of 0.1 to 2.0 mm as impregnated with the acidic composition (A). If the thickness is less than 0.1 mm, the whole acidic composition (A) is transformed into a solid gel by the solidifying/gelling agent (B), so that the absorbent loses adhesiveness to skin. This may cause insufficient wrinkle-flattening effect. If the thickness exceeds 2.0 mm, the solidifying/gelling agent (B) may not be able to accomplish sufficient solidification so that the adequate wrinkle-flattening effect is not exerted.

The size of the absorbent is properly decided according to the purpose. In a case where facial wrinkles at the outer corners of the eyes and on the forehead are to be reduced at a time, for example, the absorbent has such a size as to be able to cover substantially the overall face. In a case where facial wrinkles at a part of the facial area, such as the outer corners of the eyes or the forehead, are to be reduced, the absorbent may have such a size as to cover the target part.

It is noted that the acidic composition (A) and the solidifying/gelling agent (B) are composed substantially the same way as those of the aforementioned antipruritic agent for external use.

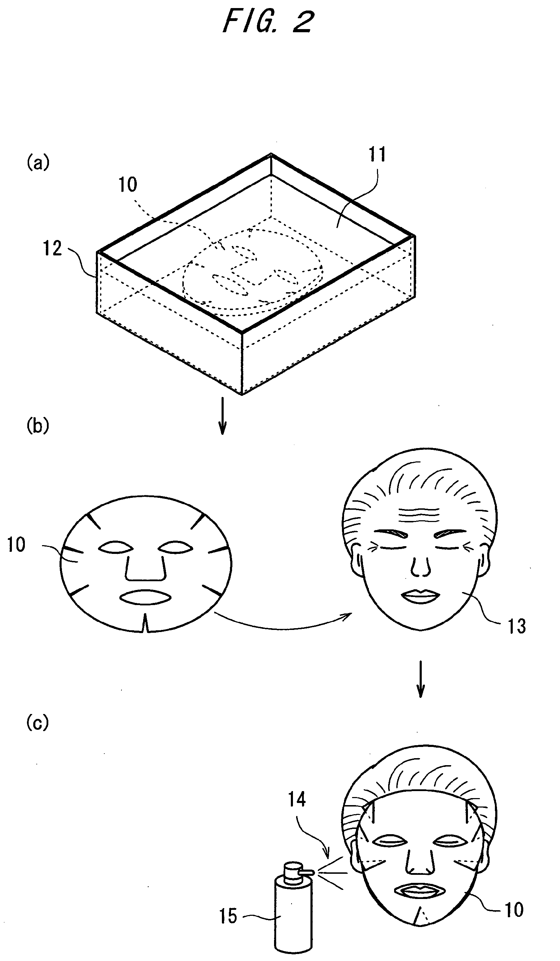

The wrinkle-flattening instrument according to the present embodiment may be used in the following manner, for example. Specifically, as shown in FIG. 2, an absorbent (face mask) 10 capable of substantially covering the overall face (excluding the eyes and mouth) is first prepared. The face mask is placed in a container 12 containing therein an acidic composition (A) 11 in liquid form, so as to uniformly impregnate the face mask 10 with the acidic composition (A) 11 (see FIG. 2A). An openable slit is formed substantially in the center of the face mask 10 such as to allow breathing through nose. Furthermore, the face mask 10 is further formed with slits at an edge thereof for tightly fitting to the face. Subsequently, the face mask 10 in the moisturized state as impregnated with the acidic composition (A) 11 is applied onto a face 13 with wrinkles at the outer corners of the eyes and on the forehead (see FIG. 2B). Then, a solidifying/gelling agent (B) 14 is supplied onto the face mask 10 impregnated with the acidic composition (A) 11 by spraying the agent from a spray can 15 (see FIG. 2C). Thus, the surfaces contacting with the wrinkled areas of the face 13 are retained in the moisturized state, while a solid gel is quickly formed on the surface of the face mask. As a result, the wrinkles can be reduced. Furthermore, if there is itching sensation at an area under the face mask, the itching sensation can be ceased because the aforementioned antipruritic agent for external use is used. In addition, an alginate(s) as the acidic polysaccharide and at least one weak acid selected from among phosphoric acid-type weak acids and ethylenediaminetetraacetic acid-type weak acids are contained in the face mask, so that the aesthetic effects such as whitening can also be obtained.

The impregnation of the acidic composition (A) is not limited to the above method. For instance, the acidic composition (A) may be sprayed onto the overall area of the face mask 10. This approach offers an advantage of omitting the aforesaid container 12.

The supply of the solidifying/gelling agent (B) is not limited to the above method. For instance, the solidifying/gelling agent (B) may be spread on the overall surfaces of both palms, which may be brought into contact with the surface of the face mask 10 in a manner to cover the mask. Alternatively, the solidifying/gelling agent (B) may be impregnated into a piece of unwoven fabric, which may be brought into contact with the face mask 10 for transferring the solidifying/gelling agent (B) into the mask.

In such uses, the using ratio between the acidic composition (A) and the solidifying/gelling agent (B) is not particularly limited so long as a sufficient amount of solidifying/gelling agent (B) is delivered to the acidic composition (A) adhered to the skin surface.

While the foregoing description pertains to the method wherein the absorbent capable of substantially covering the overall face is used, the same procedure may also be taken in a case where an absorbent of a small area is used for flattening wrinkles at a part of the face area such as the outer corners of the eyes or the forehead.

The wrinkle-flattening instrument according to the present embodiment has a primary object to obtain the wrinkle-flattening effect. Therefore, the instrument preferably adopts the combination of an aqueous solution, as the acidic composition (A), containing an alkaline metal salt of alginic acid (particularly, sodium alginate, potassium alginate), the weak acid(s) of phosphoric acid-type weak acids (particularly sodium dihydrogen phosphate, potassium dihydrogen phosphate) and water; an aqueous solution of calcium chloride or a solution mixture of calcium glycerophosphate and lactic acid, as the solidifying/gelling agent (B); and unwoven rayon fabric or unwoven cotton fabric as the absorbent.

The wrinkle-flattening instrument may be a set of packages discretely containing the acidic composition (A), the solidifying/gelling agent (B) and the absorbent, for example. Alternatively, the acidic composition (A) may previously be impregnated into the absorbent and the absorbent impregnated with the acidic composition (A) may be packed in a sealing bag (such as a bag preventing the acidic composition (A) from being dried). This package may be combined with a package of the solidifying/gelling agent (B) to constitute one set.

While the invention will be described in more details by way of reference to examples and comparative examples thereof, the invention is not limited to these examples. It is noted that Examples 1 to 14, 22 to 25 are directed to the validation of the antipruritic effect and that Examples 15 to 21, 26, 27 are directed to the validation of the wrinkle-flattening effect.

### Example 1

### Preparation of Acidic Composition

An acidic composition was prepared using 1 part by weight (hereinafter, abbreviated as "part") of sodium alginate as the alginate; 1 part of sodium dihydrogen phosphate as the weak acid, 1.5 parts of sodium carboxymethyl cellulose as the adhesive, 96.4 parts of purified water as the water and 0.1 part of methylparaben as the preservative.

### Preparation of Solidifying/Gelling Agent

A solidifying/gelling agent in liquid form was prepared using 0.5 parts of calcium chloride as the calcium salt and 99.5 parts of purified water as the water.

The resultant acidic composition and solidifying/gelling agent were combined to provide an antipruritic agent for external use.

### Example 2

### Preparation of Acidic Composition

An acidic composition was prepared using 2 parts of sodium alginate as the alginate, 1.5 parts of sodium dihydrogen phosphate as the weak acid, 96.4 parts of purified water as the water and 0.1 part of methylparaben as the preservative.

### Preparation of Solidifying/Gelling Agent

A solidifying/gelling agent in liquid form was prepared using 2 parts of calcium chloride as the calcium salt and 98 parts of purified water as the water.

The resultant acidic composition and solidifying/gelling agent were combined to provide an antipruritic agent for external use.

### Example 3

### Preparation of Acidic Composition

An acid composition was prepared using 3 parts of sodium alginate as the alginate, 2 parts of potassium dihydrogen phosphate as the weak acid, 94.9 parts of purified water as the water and 0.1 part of methylparaben as the preservative.

### Preparation of Solidifying/Gelling Agent

A solidifying/gelling agent in liquid form was prepared using 5 parts of calcium chloride as the calcium salt and 95 parts of purified water as the water.

The resultant acidic composition and solidifying/gelling agent were combined to provide an antipruritic agent for external use.

### Example 4

### Preparation of Acidic Composition

An acidic composition was prepared using 5 parts of sodium alginate as the alginate, 3 parts of sodium dihydrogen phosphate as the weak acid, 91.9 parts of purified water as the water and 0.1 part of methylparaben as the preservative.

### Preparation of Solidifying/Gelling Agent

A solidifying/gelling agent in liquid form was prepared using 10 parts of calcium chloride as the calcium salt and 90 parts of purified water as the water.

The resultant acidic composition and solidifying/gelling agent were combined to provide an antipruritic agent for external use.

### Example 5

### Preparation of Acidic Composition

An acidic composition was prepared using 1 part of potassium alginate as the alginate, 1 part of potassium dihydrogen phosphate as the weak acid, 2 parts of methyl cellulose as the adhesive, 95.9 parts of purified water as the water and 0.1 part of methylparaben as the preservative.

### Preparation of Solidifying/Gelling Agent

An acidic solidifying/gelling agent in liquid form (with partially precipitated powder) was prepared using 5 parts of calcium phosphate as the calcium salt, 1 part of citric acid as the acid and 94 parts of purified water as the water.

The resultant acidic composition and solidifying/gelling agent were combined to provide an antipruritic agent for external use.

### Example 6

### Preparation of Acidic Composition

An acidic composition was prepared using 4 parts of potassium alginate as the alginate, 2 parts of sodium dihydrogen phosphate as the weak acid, 93.9 parts of purified water as the water and 0.1 part of methylparaben as the preservative.

### Preparation of Solidifying/Gelling Agent

An acidic solidifying/gelling agent in liquid form was prepared using 5 parts of calcium lactate as the calcium salt, 5 parts of malic acid as the acid and 90 parts of purified water as the water.

The resultant acidic composition and solidifying/gelling agent were combined to provide an antipruritic agent for external use.

### Example 7

### Preparation of Acidic Composition

An acidic composition was prepared using 1 part of potassium alginate as the alginate, 1 part of disodium ethylenediaminetetraacetate as the weak acid, 97.9 parts of purified water as the water and 0.1 part of methylparaben as the preservative.

### Preparation of Solidifying/Gelling Agent

A solidifying/gelling agent in liquid form was prepared using 2 parts of calcium chloride as the calcium salt and 98 parts of purified water as the water.

The resultant acidic composition and solidifying/gelling agent were combined to provide an antipruritic agent for external use.

### Example 8

The acidic composition and solidifying/gelling agent prepared in Example 1, and a piece of unwoven rayon fabric (8 cm × 8 cm × thickness 0.3 mm) as the absorbent were combined to provide an antipruritic material for external use.

### Example 9

The acidic composition and solidifying/gelling agent prepared in Example 2, and a piece of unwoven rayon fabric (8 cm × 8 cm × thickness 0.3 mm) as the absorbent were combined to provide an antipruritic material for external use.

### Example 10

The acidic composition and solidifying/gelling agent prepared in Example 3, and a piece of unwoven rayon fabric (8 cm × 8 cm × thickness 0.3 mm) as the absorbent were combined to provide an antipruritic material for external use.

### Example 11

The acidic composition and solidifying/gelling agent prepared in Example 4, and a piece of unwoven rayon fabric (8 cm × 8 cm × thickness 0.3 mm) as the absorbent were combined to provide an antipruritic material for external use.

### Example 12

The acidic composition and solidifying/gelling agent prepared in Example 5, and a piece of unwoven rayon fabric (8 cm × 8 cm × thickness 0.3 mm) as the absorbent were combined to provide an antipruritic material for external use.

### Example 13

The acidic composition and solidifying/gelling agent prepared in Example 6, and a piece of unwoven rayon fabric (8 cm × 8 cm × thickness 0.3 mm) as the absorbent were combined to provide an antipruritic material for external use.

### Example 14

The acidic composition and solidifying/gelling agent prepared in Example 7, and a piece of unwoven rayon fabric (8 cm × 8 cm × thickness 0.3 mm) as the absorbent were combined to provide an antipruritic material for external use.

### Example 15

The acidic composition and solidifying/gelling agent prepared in Example 1, and a face mask of unwoven rayon fabric (thickness 0.3 mm) as the absorbent were combined to provide a face mask-type wrinkle-flattening instrument.

### Example 16

The acidic composition and solidifying/gelling agent prepared in Example 2, and a face mask of unwoven rayon fabric (thickness 0.3 mm) as the absorbent were combined to provide a face mask-type wrinkle-flattening instrument.

### Example 17

The acidic composition and solidifying/gelling agent prepared in Example 3, and a face mask of unwoven rayon fabric (thickness 0.3 mm) as the absorbent were combined to provide a face mask-type wrinkle-flattening instrument.

### Example 18

The acidic composition and solidifying/gelling agent prepared in Example 4, and a face mask of unwoven rayon fabric (thickness 0.3 mm) as the absorbent were combined to provide a face mask-type wrinkle-flattening instrument.

### Example 19

The acidic composition and solidifying/gelling agent prepared in Example 5, and a face mask of unwoven rayon fabric (thickness 0.3 mm) as the absorbent were combined to provide a face mask-type wrinkle-flattening instrument.

### Example 20

The acidic composition and solidifying/gelling agent prepared in Example 6, and a face mask of unwoven rayon fabric (thickness 0.3 mm) as the absorbent were combined to provide a face mask-type wrinkle-flattening instrument.

### Example 21

The acidic composition and solidifying/gelling agent prepared in Example 7, and a face mask of unwoven rayon fabric (thickness 0.3 mm) as the absorbent were combined to provide a face mask-type wrinkle-flattening instrument.

### Example 22

### Preparation of Acidic Composition

An acidic composition was prepared using 1.5 parts of sodium alginate as the alginate, 1.5 parts of sodium dihydrogen phosphate as the weak acid, 1 part of sodium carboxymethyl cellulose as the adhesive, 95.9 parts of purified water as the water and 0.1 part of methylparaben as the preservative.

### Preparation of Solidifying/Gelling Agent

An acidic solidifying/gelling agent in liquid form was prepared using 4 parts of calcium glycerophosphate as the calcium salt, 1 part of lactic acid as the acid, 90.9 parts of purified water as the water, 5 parts of glycerin as another ingredient and 0.1 part of methylparaben as the preservative.

The resultant acidic composition and solidifying/gelling agent were combined to provide an antipruritic agent for external use.

### Example 23

### Preparation of Acidic Composition

An acidic composition was prepared using 2 parts of sodium alginate as the alginate, 2 parts of potassium dihydrogen phosphate as the weak acid, 1.5 parts of sodium carboxymethyl cellulose as the adhesive, 94.4 parts of purified water as the water and 0.1 part of methylparaben as the preservative.

### Preparation of Solidifying/Gelling Agent

An acidic solidifying/gelling agent in liquid form was prepared using 3 parts of calcium glycerophosphate as the calcium salt, 1 part of sodium ethylenediaminetetraacetate as the acid, 90.9 parts of purified water as the water, 5 parts of glycerin as another ingredient and 0.1 part of methylparaben as the preservative.

The resultant acidic composition and solidifying/gelling agent were combined to provide an antipruritic agent for external use.

### Example 24

The acidic composition and solidifying/gelling agent prepared in Example 22, and a piece of unwoven cotton fabric (8 cm × 8 cm × thickness 0.3 mm) as the absorbent were combined to provide an antipruritic material for external use.

### Example 25

The acidic composition and solidifying/gelling agent prepared in Example 23, and a piece of unwoven cotton fabric (8 cm × 8 cm × thickness 0.3 mm) as the absorbent were combined to provide an antipruritic material for external use.

### Example 26

The acidic composition and solidifying/gelling agent prepared in Example 22, and a face mask of unwoven cotton fabric (thickness 0.3 mm) as the absorbent were combined to provide a face mask-type wrinkle-flattening instrument.

### Example 27

The acidic composition and solidifying/gelling agent prepared in Example 23, and a face mask of unwoven cotton fabric (thickness 0.3 mm) as the absorbent were combined to provide a face mask-type wrinkle-flattening instrument.

### Comparative Example 1

According to Example 1 of Japanese Unexamined Patent Publication No. 11-178910, 1 (weight/volume)% solution of sodium alginate, and 5 (weight/volume)% solution of calcium chloride were prepared.

### Comparative Example 2

The sodium alginate solution and calcium chloride solution prepared in Comparative Example 1, and a face mask of unwoven rayon fabric (thickness 0.3 mm) as the absorbent were prepared.

### Comparative Example 3

### Preparation of Acidic Composition

An acidic composition was prepared using 1 part of sodium alginate as the alginate, 1 part of succinic acid as the acid, 97.9 parts of purified water as the water and 0.1 part of methylparaben as the preservative.

### Preparation of Solidifying/Gelling Agent

A solidifying/gelling agent in liquid form was prepared using 2 parts of calcium chloride as the calcium salt and 98 parts of purified water as the water.

The resultant acidic composition and solidifying/gelling agent were combined to provide an antipruritic agent for external use for comparison.

### Evaluation Test

The test samples of the examples and comparative examples thus obtained were subjected to the following evaluations.

### Evaluation 1: Effect on Pruritus Associated with Insect Bites

0.3 ml of acidic composition of Example 2 was painted on a mosquito bite area on the dorsum of the left hand of a 13-year-old boy (coating thickness of about 0.5 mm) and then, 0.1 ml of solidifying/gelling agent of Example 2 was sprayed onto the coating. The acidic composition was immediately solidified while the itching sensation ceased. On the other hand, 0.3 ml of sodium alginate solution of Comparative Example 1 was painted on a mosquito bite area on the dorsum of the right hand (coating thickness of about 0.5 mm) and then, 0.1 ml of calcium chloride solution of Comparative Example 1 was sprayed onto the coating. Although the sodium alginate solution was immediately solidified, the antipruritic effect was not confirmed.

### Evaluation 2: Effect on Pruritus Associated with Eczema

2 ml of acidic composition of Example 3 was painted on an eczema area on the right thigh of a 9-year-old girl (coating thickness of about 0.5 mm) and then, 0.2 ml of solidifying/gelling agent of Example 3 was sprayed onto the coating. The acidic composition was immediately solidified while the itching sensation ceased. On the other hand, 2ml of sodium alginate solution of Comparative Example 1 was painted on an eczema area on the left thigh (coating thickness of about 0.5 mm) and then, 0.2 ml of calcium chloride solution of Comparative Example 1 was sprayed onto the coating. Although the sodium alginate solution was immediately solidified, the antipruritic effect was not confirmed.

### Evaluation 3: Effect on Pruritus Associated with Heat Rash

1 ml of acidic composition of Example 4 was painted on a heat rash area on the nose of a 47-year-old man (coating thickness of about 0.5 mm) and then, 0.1 ml of solidifying/gelling agent of Example 4 was sprayed onto the coating. The acidic composition was immediately solidified while the itching sensation ceased.

### Evaluation 4: Effect on Senile Pruritus

Two pieces of unwoven fabric impregnated with 6 ml of the acidic composition of Example 9 (3 ml per piece) was applied to a senile pruritus area on the right foot of a 71-year-old man and then, 0.4 ml of solidifying/gelling agent of Example 9 was sprayed onto each of the fabric pieces. The acidic composition impregnated into the unwoven fabric was immediately solidified while the itching sensation ceased.

### Evaluation 5: Effect on Pruritus

0.5 ml of acidic composition of Example 7 was painted on an itching area of unknown cause on the right palm of a 9-year-old girl (coating thickness of about 0.5 mm) and then, 0.2 ml of solidifying/gelling agent of Example 7 was sprayed onto the coating. The acidic composition was immediately solidified while the itching sensation ceased. On the other hand, 0.5 ml of acidic composition of Comparative Example 3 was painted on an itching area of unknown cause on the left palm (coating thickness of about 0.1 mm) and then, 0.2 ml of solidifying/gelling agent of Comparative Example 3 was sprayed onto the coating. Although the acidic composition was immediately solidified, the itching sensation did not cease. Hence, the left palm was washed. Subsequently, 0.5 ml of acidic composition of Example 2 was painted on the itching area of unknown cause on the left palm (coating thickness of about 0.1 mm) and then, 0.2 ml of solidifying/gelling agent of Example 2 was sprayed onto the coating. The acidic composition was immediately solidified while the itching sensation ceased. What is more, Example 2 exerted a greater antipruritic effect than Example 7.

### Evaluation 6: Effect of Flattening Facial Wrinkles at Outer Corners of Eyes and on Forehead

A test was conducted by female monitors aged 27 to 42. Face masks of unwoven fabric were each impregnated with 15 ml of acidic composition of each of Examples 15 to 21, and a face mask of unwoven fabric was impregnated with 15 ml of sodium alginate solution of Comparative Example 2. Each monitor tested a total of 7 face masks of the examples and the comparative example on a one-mask-per-example basis. Specifically, on any day, each monitor applied any one of the face masks onto her face and sprayed 0.2 ml of solidifying/gelling agent of each corresponding example or of calcium chloride solution of Comparative Example 2 onto the mask. With the wrinkle-flattening instrument of Examples 15 to 21 wherein the individual acidic compositions were immediately solidified, all the monitors felt the face masks tightly fitted on their faces while feeling their faces tightened. In Comparative Example 2, on the other hand, the sodium alginate solution was also immediately solidified but the monitors felt the face masks loosely fitted on their faces while feeling their faces tightened weakly. After the lapse of 5 minutes, the face masks were removed. The monitors using the wrinkle-flattening instruments of Examples 15 to 21 found that the facial wrinkles at the outer corners of the eyes and on the forehead were removed. In addition, a dramatic whitening effect to impart skin clearness was noted. Particularly, a monitor complaining of itching sensation associated with shampoo contact dermatitis on the forehead reported that the wrinkle-flattening instruments of all the examples offered advantage in ceasing the itching sensation immediately. In contrast, the instrument of Comparative Example 2 did not exert the antipruritic effect or the wrinkle-flattening effect.

While a particularly specific description is not given, it was confirmed that the antipruritic agents for external use of Examples 1, 5, 6 exerted as much antipruritic effect as those of Examples 2, 3, 4, 7, and that the antipruritic materials for external use of Examples 8, 10, 11,12, 13, 14 exerted as much antipruritic effect as the material of Example 9.

### Evaluation 7: Effect on Eczema-Related Pruritus

1 ml of acidic composition of Example 22 was painted on a half of an eczema area on the right inner arm of a 10-year-old girl (coating thickness of about 0.1 mm) and then, 0.2 ml of solidifying/gelling agent of Example 22 was sprayed onto the coating. The acidic composition was immediately solidified while the itching sensation ceased. The above solidifying/gelling agent was sprayed onto the remaining half of the eczema area that was free from the above acidic composition. The agent caused no irritations such as pain. Furthermore, the above solidifying/gelling agent did not taste bitter to the mouth nor caused so much irritation in the eyes.

While a particularly specific description is not given, the antipruritic agent for external use of Example 23 exerted as much antipruritic effect as that of Example 22. It was also confirmed that the agent caused no irritations such as pain.

Further, the antipruritic materials for external use of Examples 24, 25 were confirmed to exert as much antipruritic effect as that of Example 22. It was also confirmed that the wrinkle-flattening instruments of Examples 26, 27 exerted as much wrinkle-flattening effect as that of Example 15.

### Industrial Applicability

As described above, the skin composition for external use of the present invention employs an alginate(s) and at least one weak acid selected from among phosphoric acid-type weak acids and ethylenediaminetetraacetic acid-type weak acids. Therefore, the composition quickly forms a solid gel, providing the immediate antipruritic effect.

Furthermore, the aesthetic effects such as whitening can also be obtained because an alginate(s) as the acidic polysaccharide component and the weak acid of phosphoric acid-type weak acids or ethylenediaminetetraacetic acid-type weak acids as the acid component are used. According to the skin composition for external use of the present invention employing an absorbent in addition to the acidic composition (A) and the solidifying/gelling agent (B), the skin wrinkles can be readily flattened because the absorbent is used for forming a solid gel. In addition, the immediate antipruritic effect and the aesthetic effects such as whitening can also be provided. Therefore, the skin composition for external uses of the present invention can preferably be used as cosmetics, medicinal products, quasi drugs and the like.

## Claims

1. A skin composition for external use comprising: the following acidic composition (A); and the following solidifying/gelling agent (B) which is to be supplied to the acidic composition (A) sticking to the skin:
(A) an acidic composition (A) containing: an alginate(s), at least one weak acid selected from among phosphoric acid-type weak acids and ethylenediaminetetraacetic acid-type weak acids, and water;
(B) a solidifying/gelling agent containing a calcium salt(s).

2. The skin composition for external use according to Claim 1,
wherein the alginate is at least one selected from among sodium alginate and potassium alginate.

3. The skin composition for external use according to Claim 1,
wherein the weak acid of phosphoric acid-type weak acids is at least one selected from among sodium dihydrogen phosphate and potassium dihydrogen phosphate.

4. The skin composition for external use according to Claim 1,
wherein the calcium salt is at least one selected from among calcium chloride and calcium glycerophosphate.

5. The skin composition for external use according to Claim 1,
wherein the solidifying/gelling agent is an aqueous solution containing calcium glycerophosphate and lactic acid.

6. The skin composition for external use according to Claim 1,
wherein the acidic composition (A) further contains an adhesive for enhancing affinity for skin surface.

7. The skin composition for external use according to Claim 1,
further comprising an absorbent which is to be impregnated with the acidic composition (A) and which is to be directly applied onto skin surface as impregnated with the acidic composition (A) thereby making the acidic composition (A) adhered to the skin surface.

8. A face mask-type skin composition for external use according to Claim 7,
wherein the absorbent is shaped like a face mask substantially covering the overall facial area.

9. An antipruritic agent for external use comprising the skin composition for external use according to any one of Claims 1 to 6.

10. A wrinkle-flattening instrument comprising the skin composition for external use according to Claim 7.

11. A face mask-type wrinkle-flattening instrument comprising the face mask-type skin composition for external use according to Claim 8.
